(19) Europäisches Patentamt
European Patent Office
Office européen des brevets

(11) Publication number: **0 229 718 B1**

(12) **EUROPEAN PATENT SPECIFICATION**

(45) Date of publication of patent specification: **17.04.91** (51) Int. Cl.$^5$: **A61K 49/02**

(21) Application number: **87300302.4**

(22) Date of filing: **14.01.87**

(54) **Labelling blood cells.**

(30) Priority: **16.01.86 GB 8601003**

(43) Date of publication of application:
**22.07.87 Bulletin 87/30**

(45) Publication of the grant of the patent:
**17.04.91 Bulletin 91/16**

(84) Designated Contracting States:
**DE FR GB**

(56) References cited:
**EP-A- 0 123 504**
**EP-A- 0 179 608**
**EP-A- 0 194 843**

**THE LANCET, 25th October 1986, pages 946-949; A.M. PETERS et al.: "Clinical experience with 99mTc-hexamethylpropylene-amineoxime for labelling leucocytes and imaging inflammation"**

**THE LANCET, 6th July 1985, pages 50-51; P.J. ELL et al.: "Regular cerebral blood flow mapping with 99mTc-labelled compound"**

(73) Proprietor: **AMERSHAM INTERNATIONAL plc**
**Amersham Place**
**Little Chalfont Buckinghamshire HP7 9NA(GB)**

(72) Inventor: **Kelly, James Duncan**
**c/o Amersham International plc**
**Little Chalfont Bucks., HP7 9NA(GB)**
Inventor: **Neirinckx, Rudi Dominique**
**20 Elm Close**
**Armersham, Bucks(GB)**
Inventor: **Nowotnik, David Peter**
**12 Long Plough**
**Aston Clinton Bucks.(GB)**

(74) Representative: **Pennant, Pyers et al**
**Stevens, Hewlett & Perkins 5 Quality Court**
**Chancery Lane**
**London, WC2A 1HZ(GB)**

**EP 0 229 718 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

EPA 123504 describes lipophilic macrocyclic complexes of technetium-99m with ethylene or propylene amine oximes. By virtue of their zero net charge, these complexes are capable of crossing the blood brain barrier, and are of interest for in vivo studies of the brain.

Our earlier specification EPA 194843 describes a family of compounds in this field which have been found to show improved brain retention. This family of compounds consists of technetium-99m complexes of propylene amine oximes having the general structure 1, where each starred carbon atom carries a single hydrogen atom. It should be understood that each carbon atom may carry substituent groups other than hydrogen. In particular the technetium-99m complex of compound 2, which is 4,8-diaza-3,6,6,9-tetramethylundecane-2,10-dione bis oxime, has interesting brain retention properties. The d,l- form of compound 2 is being marketed commercially for labelling by the purchaser and used as a brain scanning agent indicating brain bloodflow.

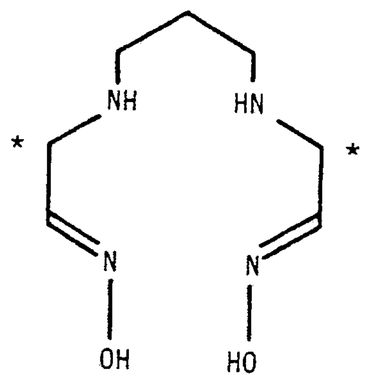

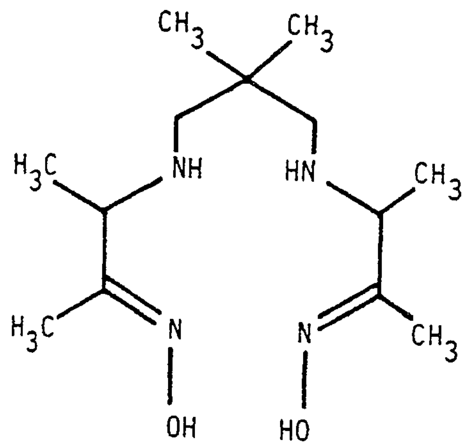

Our earlier specification EPA 179608 describes another family of compounds in this field, having a general structure as 1 but in which only one of the starred carbon atoms carries a single hydrogen, and which are therefore asymmetric about the 5-carbon atoms.

This invention results from the discovery that technetium-99m complexes of compounds of structure 1 and related compounds are valuable agents for labelling blood cells. This discovery was unexpected. The prior literature in this field gives little reason for connecting brain scanning agents and brain bloodflow agents on the one hand with blood cell labelling agents on the other hand.

The conventional technique for labelling blood cells involves extracting a sample of blood from the patient, recovering the desired cells in relatively pure form, labelling the cells in vitro, and injecting the labelled cells back into the patient. At present, the preferred blood cell labelling agent is a complex of indium-111 with oxine (8-hydroxyquinoline). But this complex, together with other complexes of indium-111, suffers from several disadvantages:-

- Cost and convenience. Indium-111 is relatively expensive and does not have an extensive range of applications; technetium-99m is cheap and useful for many other purposes.
- The radiation dose to the patient. For same day or next day scanning, a technetium-99m complex would give rise to a lower overall radiation dose.
- The intracelluar radiation dose to lymphocytes. Indium-111 generates Auger electrons which have caused concern over their potential to initiate lymphocytic leukemia.

We have calculated relative radiation doses to whole body, critical organs (liver and spleen) and to lymphocytes for a typical leucocyte labelling procedure using indium-111 versus technetium-99m. The calculations are based on accepted assumptions for the biodistribution of labelled leucocytes.

Relative doses for indium-111 and technetium-99m leucocytes

| | Dose(Rad) per millicurie | |
|---|---|---|
| | In-111 | Tc-99m |
| Whole body | 0.5 | 0.02 |
| Liver | 4.0 | 0.13 |
| Spleen | 27.0 | 0.87 |
| White cells (1mCi/3.5x$10^8$ cells) | 6000 | 280 |

It is apparent that Tc-99m is very much favoured for same day imaging. But there are also advantages for next day imaging. If one wishes to scan after 18 hours, i.e. at 3 half-lives, it is necessary to use 8 x more Tc-99m to obtain a suitable activity at the time of scanning. But Tc-99m still gives a substantially lower radiation dose.

There is a need for a blood cell labelling agent based on technetium-99m.

The invention provides a method of labelling blood cells by incubating a suspension of the blood cells with a neutral lipophilic technetium-99m complex of a propylene amine oxime having structure 2.

Although this invention is based on results and not on theories, we currently believe that the technetium-99m complex of this compound may be prone to decompose in situ in blood cells in such a way as to retard or prevent its elution.

A blood cell suspension is incubated with a complex as defined above under conditions which are not very critical. Incubation temperature may be from 0 to 50°C and is typically ambient or 37° C. Incubation time is typically from 1 to 60 minutes, although under favourable conditions labelling is substantially complete within ten minutes. Blood cells which may be labelled by the method include erythrocytes, leucocytes both mononuclear and polynuclear, and platelets. The efficiency of the method depends on the nature of the cells, and is generally better for leucocytes than for erythrocytes, and better for erythrocytes than for platelets. The concentration of the cells in the suspension is preferably as high as possible. As far as leucocytes are concerned, a substantial uptake of complex is observed at concentrations of $10^7$ cells/ml and greater, while at concentrations below $10^6$ cells/ml, insufficient uptake is observed. Similar effects apply for other kinds of blood cells. Plasma inhibits labelling. For example in one experiment using human erythrocytes, labelling was 50% inhibited by a final concentration of 16.9% plasma and 100% inhibited by 34% plasma. In cases when cells have been separated from blood for labelling, plasma should be substantially absent. On the other hand, leucocytes and particularly platelets may require plasma in order to remain viable. Plasma concentrations should be chosen sufficiently high to maintain blood cell viability during labelling but sufficiently low to permit efficient labelling. For leucocytes and other blood cells, plasma concentrations from 5% to 20% may be appropriate. Indeed, addition of plasma is often a convenient way of terminating the labelling reaction. Only with whole blood labelling, where mixed blood cells are present in very high concentration, is the presence of substantial proportions of plasma acceptable.

The technetium-99m complexes are eluted from the blood cells into which they have been absorbed, but generally at a rate which is slow compared to the half life of technetium-99m (6 hours). The rate of elution is markedly temperature dependent, so labelled blood cells should preferably be stored at ambient temperature or below prior to use. The nature of the blood cells is also important, as indicated by the following figures which relate to different kinds of labelled cells stored for different periods of time in whole blood.

| Cell Type | Storage Period (hours) | Temperature ($^oC$) | % Radioactivity (not cell-bound) |
|---|---|---|---|
| Platelets | 10 | 20 | 23 |
| Platelets | 20 | 4 | 28 |
| Erythrocytes | 20 | 20 | 13 |
| Leucocytes | 20 | 20 | 13 |
| Leucocytes | 20 | 37 | 28 |

The following examples illustrate the invention.

Example 1

A solution of the technetium-99m complex of compound 2 was made as follows. A vial contained 0.5mg compound 2; 0.0075mg stannous chloride dihydrate; and 4.5mg sodium chloride; freeze-dried and sealed under nitrogen. To this was added 5.0ml of sodium pertechnetate eluate from a 135mCi technetium generator.

Mixed leucocytes were obtained from 34ml of acid citrate anticoagulated blood by dextran sedimentation. These were washed twice and resuspended in 2.0ml phosphate buffered saline. The suspension was incubated with 0.2ml of the solution of the technetium-99m complex of compound 2. Incubation was at ambient temperature, and samples were removed at intervals for analysis. After two minutes, 60% of the radioactivity was associated with blood cells; after 5 minutes, 83%; and after 10 minutes, 89%.

Example 2

To 0.2ml of the solution of the technetium-99m complex of compound 2 were added 1.0ml of washed erythrocytes at varying concentrations. Incubation was for 10 minutes at ambient temperature. With a red blood cell number of $10^8$, 30% of the radioactivity was taken up in the cells; with $10^9$ cells, the uptake was 80%; with $10^{10}$ cells, the uptake was 90%.

Example 3

The experiment of Example 2 was repeated, but using platelets in place of erythrocytes. With 2 x $10^9$ cells/ml, the uptake of radioactivity was 36%.

Example 4

1.0 ml samples of heparinized whole blood (including plasma) were incubated with from 10 to 200 microlitres of the solution of the technetium-99m complex of compound 2. Labelling efficiency increased in proportion to the amount of complex, ranging from 35.6% for a 10 microlitre addition to 68.3% for 200

microlitres.

Elution of technetium from these essentially erythrocyte preparations demonstrated that over a period of up to four hours leaching from the cells was less than 11%.

Example 5

Tc-99m labelled leucocytes prepared by the method of Example 1 were injected into rats bearing abscesses produced by implantation of sponge impregnated with faecal extract. Abscess uptake was identical to that for In-III labelled leucocytes.

## Claims

1. A method of labelling blood cells by incubating a suspension of the blood cells with a neutral lipophilic technetium-99m complex of a propylene amine oxime wherein the propylene amine oxime is 4,8-diaza-3,6,6,9-tetramethylundecane-2, 10-dione bis oxime.

2. A method as claimed in claim 1 , wherein the d,l-form of the propylene amine oxime is used.

3. A method as claimed in claim 1 wherein the suspension of blood cells is substantially free of plasma.

4. A method as claimed in any one of claims 1 to 3 wherein the blood cells are leucocytes.

5. A method as claimed in claim 4 wherein the cell concentration of the leucocyte suspension is at least $10^6$ cell s/ml.

6. A method as claimed in any one of claims 1 to 3 wherein the blood cells are erythrocytes.

## Revendications

1. Procédé de marquage de cellules sanguines par incubation d'une suspension des cellules sanguines avec un complexe lipophile neutre de technétium-99m et d'une propylène-amine-oxime, dans lequel l'oxime de propylene-amine est la bis-oxime de 4,8-diaza-3,6,6,9-tétraméthyl-undécane-2,10-dione.

2. Procédé selon la revendication 1, dans lequel on utilise la forme d,l de la propylène-amine-oxime.

3. Procédé selon la revendication 1, dans lequel la suspension de cellules sanguines est sensiblement exempte de plasma.

4. Procédé selon l'une quelconque des revendications 1 à 3, dans lequel les cellules sanguines sont des leucocytes.

5. Procédé selon la revendication 4, dans lequel la concentration cellulaire de la suspension de leucocytes est d'au moins $10^6$ cellules/ml.

6. Procédé selon l'une quelconque des revendications 1 à 3, dans lequel les cellules sanguines sont des érythrocytes.

## Ansprüche

1. Verfahren zum Markieren von Blutzellen durch Inkubieren einer Suspension der Blutzellen mit einem neutralen lipophilen Technetium-99m-Komplex eines Propylenaminoxims, worin das Propylenaminoxim ein 4,8-Diaza-3,6,6,9-tetramethylundecan-2,10-dion-bis-oxim ist.

2. Verfahren nach Anspruch 1, worin die d,l-Form des Propylenaminoxims verwendet wird.

3. Verfahren nach Anspruch 1, worin die Suspension von Blutzellen im wesentlichen frei von Plasma ist.

4. Verfahren nach einem der Ansprüche 1 bis 3, worin die Blutzellen Leukozyten sind.

5. Verfahren nach Anspruch 4, worin die Zellkonzentration der Leukozytensuspension wenigstens $10^6$ Zellen/ml beträgt.

6. Verfahren nach einem der Ansprüche 1 bis 3, worin die Blutzellen Erythrozyten sind.